(19) Europäisches Patentamt
European Patent Office
Office européen des brevets

(11) Veröffentlichungsnummer: **0 101 602**
**B1**

(12) # EUROPÄISCHE PATENTSCHRIFT

(45) Veröffentlichungstag der Patentschrift: **01.06.88**

(51) Int. Cl.⁴: **C 07 D 498/04**, C 07 D 499/06

(21) Anmeldenummer: **83108057.7**

(22) Anmeldetag: **16.08.83**

(54) **In 6-Stellung unsubstituierte 7-Oxo-4-oxa-diazabicyclo-(3.2.0)-hept-2-en Derivate, Verfahren zu ihrer Herstellung und ihre Verwendung als Zwischenprodukte zur Synthese von beta-Lactamantibiotika.**

(30) Priorität: **20.08.82 DE 3231060**

(43) Veröffentlichungstag der Anmeldung: **29.02.84 Patentblatt 84/9**

(45) Bekanntmachung des Hinweises auf die Patenterteilung: **01.06.88 Patentblatt 88/22**

(84) Benannte Vertragsstaaten: **CH DE FR GB IT LI SE**

(56) Entgegenhaltungen:
**CH - A - 566 980**
**DE - A - 2 800 860**

**JOURNAL OF THE AMERICAN CHEMICAL SOCIETY, Band 94, 1972, Seiten 1021-1022 (COOPER et al)**
**R J Stoodley, Proc. of the seventh Workshop Conf., Hoechst, SchloB Reisenburg (1978), 193-203**
**L. Ghosez et al., Tetrahedron, Vol. 39, 2493 (1963)**

(73) Patentinhaber: **BAYER AG, Konzernverwaltung RP Patentabteilung, D-5090 Leverkusen 1 Bayerwerk (DE)**

(72) Erfinder: **Häbich, Dieter, Dr., Birkenhöhe 2, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft neue, am 6-Stickstoffatom unsubstituierte 7-Oxo-4-oxa-2,6-diazabicyclo[3.2.0]hept-2-en-Derivate, mehrere Verfahren zu ihrer Herstellung und ihre Verwendung als Zwischenprodukte insbesondere zur Synthese von β-Lactamantibiotika.

Es ist bereits bekannt geworden, dass N-substituierte Oxazolinoazetidinone durch intramolekulare Cyclisierung zum stereoselektiven Aufbau von 1-Oxadethia-3-cephem-4-carbonsäure geeignet sind (T. Aoki et al. Heterocycles 15 (1981) 409; T. Aoki et al. Tetrahedron Lett. (1979) 4327; Deutsche Offenlegungsschriften 2 806 457 und 2 800 860).

In der CH-PS 566 980 und in J. Am. Chem. Soc. Bd. 94, 1972, Seiten 1021–1022 (Cooper et al.) werden Thiazolidinazetidinone beschrieben, die am Stickstoff unsubstituiert sind.

Ferner ist bekannt, dass am Stickstoffatom unsubstituierte Azetidinone bereitwillig mit Elektrophilen verschiedener Art zu reagieren vermögen (z.B. R.B. Woodward et al. J. Am. Chem. Soc. 88 (1966) 852; FR-A-1 495 047).

Unter diesen Aspekten würden am Stickstoffatom unsubstituierte Oxazolinoazetidinone sehr wünschenswerte Verbindungen zur Herstellung von β-Lactamantibiotika darstellen. Ihre Herstellung und Isolierung wurden jedoch bisher vergeblich versucht (R.J. Stoodley, Proc. of the Seventh Workshop Conf. Hoechst, Schloss Reisensburg, (1978) 193–203, R.J. Stoodley in J. Elks, Recent Advances in the Chemistry of β-Lactam Antibiotics, The Chemical Society London (1977), 189.

Die vorliegende Erfindung stellt nun Oxazolinoazetidinone der allgemeinen Formel (1)

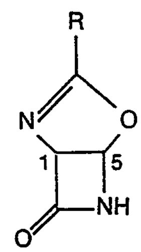

(1)

in welcher

R für einen in β-Lactamantibiotika üblichen Rest aus der Gruppe bestehend aus Wasserstoff, gegebenenfalls substituiertes Alkyl, Alkenyl, Alkinyl, Aralkyl, mit der Ausnahme unsubstituiertes Benzyl, Aryl, Heteroaryl, Heteroaralkyl, Aryloxyalkyl, Heteroaryloxyalkyl, Alkoxyalkyl, Arylthioalkyl, Heteroarylthioalkyl, Alkylthioalkyl, Alkoxy, Aryloxy, Alkylthio oder Arylthio steht, zur Verfügung.

Diese neuen, am Stickstoffatom unsubstituierten Verbindungen besitzen den Vorzug, weitaus flexibler als die benannten N-substituierten Derivate, sowohl mit mono- als auch bifunktionellen Reagentien zu β-Lactamantibiotika umsetzen zu lassen. Eine derartige Synthese von β-Lactamantibiotika ist konvergent und damit effektiv. Durch den nicht von vornherein anwesenden N-Substituenten wird das Spektrum der möglichen Zielverbindungen entscheidend erweitert.

Die erfindungsgemässen Verbindungen können dadurch erhalten werden, dass man

(a) N-substituierte Oxazolinoazetidinone der allgemeinen Formel (2)

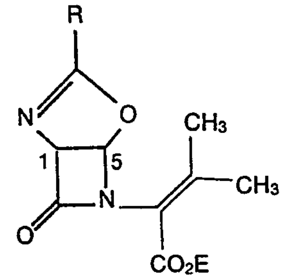

(2)

in welcher

R die oben angegebene Bedeutung hat und

$CO_2E$ für eine Säurefunktion $CO_2H$ oder eine beliebige Esterfunktion steht, wobei E für die in der β-Lactamchemie üblicherweise verwendeten Säureschutzgruppen steht, vorzugsweise für einen $C_1$–$C_4$-Alkylrest steht, mit einem Oxidationsmittel unter solvolytischen Bedingungen in einem geeigneten Lösungsmittel oder Lösungsmittelgemisch, gegebenenfalls in Gegenwart von säurebindenden Mitteln oder anschliessendem Einsatz von Reduktionsmitteln umsetzt oder

(b) Oxamide der allgemeinen Formel (3)

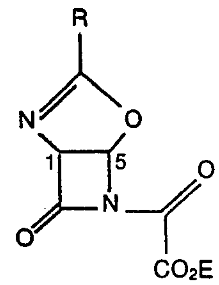

(3)

in welcher

R und $CO_2E$ die oben angegebene Bedeutung haben, unter solvolytischen Bedingungen in einem geeigneten Lösungsmittel oder Lösungsmittelgemisch, gegebenenfalls in Gegenwart von Säuren oder Basen umsetzt.

Die Verbindungen der allgemeinen Formel (2), in welcher R und $CO_2E$ die angegebene Bedeutung haben, können in Analogie zu bekannten Verfahren dadurch erhalten werden, dass man die olefinische Bindung von Verbindungen der allgemeinen Formel (4)

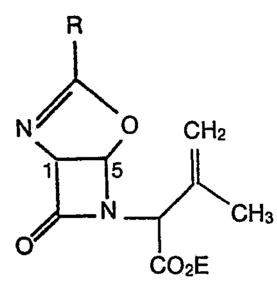

(4)

in welcher

R und $CO_2E$ die oben angegebene Bedeutung haben, in einem geeigneten Lösungsmittel in Gegenwart von Base einer Isomerisierung unterwirft, wie sie beispielsweise bei Y. Maki et al.,

J.C.S. Perkin I (1981) 2087, Y. Hamashima et al., Tetrahedron Lett. (1979) 2595, S. Uyeo et al., J. Am. Chem. Soc. 101, 4403 (1979) und in der BE-B 862 793 beschrieben ist.

Die Oxazolinooxamide der allgemeinen Formel (3) können ebenfalls in Analogie zu bekannten Verfahren dadurch erhalten werden, dass man die olefinische Bindung von Azetidinonen der allgemeinen Formel (2), in welcher R und $CO_2E$ die angegebene Bedeutung haben, einer oxidativen Spaltung unterwirft wie sie beispielsweise in der DE-A-2 839 646, EP-B-21 676, der BE-B-849 118 und bei S. Yamamoto et al. Heterocycles 8, 282 (1977) und M. Narisada et al. J. Med. Chem. 22, 757 (1979), Heterocycles 7, 839 (1977) in analoger Weise beschrieben ist.

Ausserdem können die Verbindungen der allgemeinen Formel (2) auch in Analogie zu anderen literaturbekannten Verfahren hergestellt werden, z. B. dem in der Japanischen Patentschrift 55 047 687, der Niederländischen Patentschrift 7 313 896 oder bei Y. Hamashima et al., Tetrahedron Lett. (1979) 4943 beschrieben.

Als mögliche Ausgangsprodukte für optisch aktive Oxazolinoazetidinone der allgemeinen Formel (1S,5R) (1) und (1R,5S) (1) werden gegebenenfalls die entsprechend konfigurierten Verbindungen der allgemeinen Formel (4) verwendet.

Für die Synthese beider Enantiomere der Verbindung der allgemeinen Formel (4), in der R = Phenyl ist, existieren Literaturbeispiele:

(1S,5R) (4): S. Yamamoto et al., Tetrahedron Lett. (1981) 3089.

(1R,5S) (4): Y. Hamashima et al., Tetrahedron Lett. (1979) 2595.

Andere Verbindungen (4) können in analoger Weise hergestellt werden.

Verwendet man z. B. Diphenylmethyl-2-[(1S,5R)-3-phenyl-7-oxo-4-oxa-2,6-diazabicyclo[3.2.0]hept-2-en-6-yl]-3-methyl-but-3-enoat (4a), so kann der Reaktionsablauf zur Herstellung der erfindungsgemässen Verbindungen (1) durch das folgende Formelschema wiedergegeben werden:

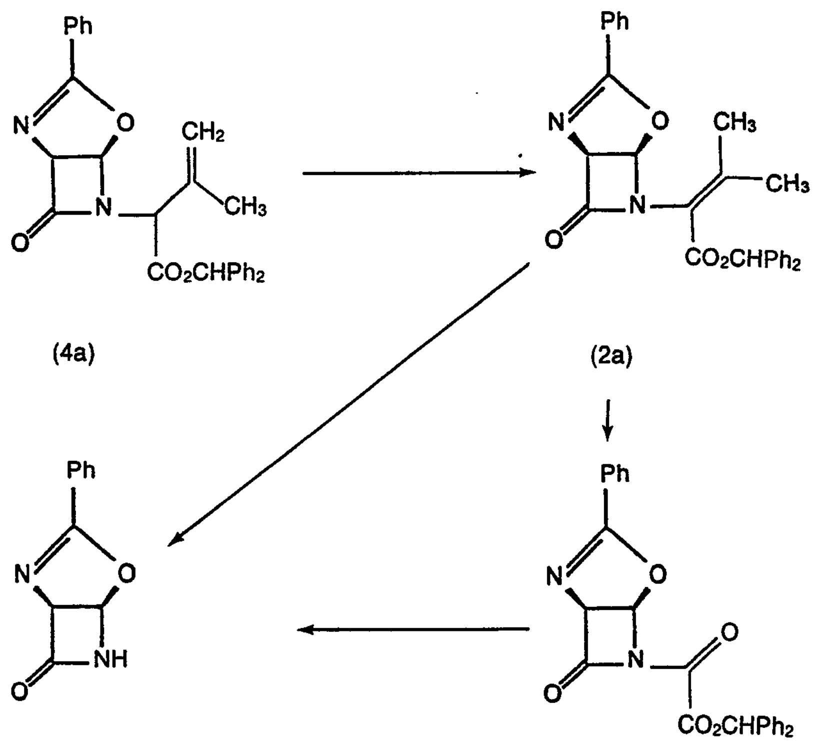

(4a) (2a)

(1a) (3a)

In den Verbindungen der allgemeinen Formel (1), (2), (3) und (4) steht für gegebenenfalls substituiertes Alkyl ein geradkettiger, verzweigter oder cyclischer Kohlenwasserstoffrest mit vorzugsweise 1–7 C-Atomen. Die Alkylreste können gegebenenfalls ungesättigt vorliegen und durch Halogen vorzugsweise Chlor, Hydroxy, Amino, Carboxy, Carbamoyl, Mesyl oder durch im folgenden näher definiertes, gegebenenfalls substituiertes Aryl bzw. Heteroaryl, einfach oder doppelt substituiert sein. Besonders erwähnt seien hier Reste wie Methyl, Halogenmethyl, tert.-Butyl, Cyclohexyl und Cyclohexadienyl.

Für gegebenenfalls substituiertes Aryl steht vorzugsweise Phenyl, welches durch Methyl, Ethyl, Aminoethyl, Hydroxy, Methoxy, Ethoxy, Carbamoyloxy, Acetoxy, Amino, Mesylamino, Methylamino, Aminosulfonylamino, Amidino, Mesyl, Methylsulfinyl, Methoxycarbonyl, Carbamoyl, Sulfo, Methylthio, Silyl, Silyloxy oder Halogen vorzugsweise einfach oder doppelt, gegebenenfalls aber auch 3fach substituiert sein kann.

Für gegebenenfalls substituiertes Aralkyl stehen die Kombinationen der unter Aryl und Alkyl genannten Bedeutungen, mit Ausnahme unsubstituiertes Benzyl. Besonders erwähnt seien: p-Hydroxybenzyl, p-Aminobenzyl, α-Aminobenzyl, α,4-Diaminobenzyl, α-Amino-4-hydroxybenzyl, α-Carboxy-benzyl, α-Carboxy-4-hydroxybenzyl und bis-(Trimethylsilyl)-geschütztes α-Carboxy-4-hydroxybenzyl.

Für gegebenenfalls substituiertes Heteroaryl stehen alle im Ring Sauerstoff-, Stickstoff- oder Schwefelatome enthaltenden ungesättigten 5- oder 6-gliedrigen Heterocyclen mit 1–4 Heteroatomen, die unsubstituiert oder durch Methyl, Ethyl, Hydroxy, Oxo, Amino, Imino, Mesyl, Mesylamino, Silyl, Carboxy, Carbamoyl, Acetyl oder Halogen mono-, di- oder trisubstituiert sein können.

Vorzugsweise steht für einen ungesättigten gegebenenfalls substituierten heterocyclischen Ring die Furyl-, Methylfuryl-, Thienyl-, Methylthienyl-, 2-Aminothiazolyl-, Thiazolyl-, Methylisoxazolyl-, Isoxazolyl-, Pyridyl-, 2-Aminopyridyl-, Pyrimidyl-, Pyrazolyl-, Uracyl-, Thiadiazolyl-, Tetrazolyl- oder Pyranylgruppe.

Für gegebenenfalls substituiertes Heteroalkyl stehen die Kombinationen der unter Alkyl oder Heteroaryl vorzugsweise genannten Bedeutungen. Besonders erwähnt seien hier Furylmethyl, Thienylmethyl, 2-Aminothiazolylmethyl, Thiazolylmethyl, Aminopyridylmethyl, 1-Methyl-1-H-tetrazol-5-yl-thiomethyl, 2-Aminothiazolyl-methoxyiminomethyl, 1-(2-Aminothiazolyl)-1-propenyl.

Für gegebenenfalls substituiertes Aryloxyalkyl, Heteroaryloxyalkyl und Alkoxyalkyl stehen die obengenannten Bedeutungen, die im Alkylteil oder zwischen Alkyl- und Aryl- bzw. Heteroarylteil eine Sauerstoffbrücke in Form einer Etherfunktion tragen. Besonders erwähnt seien: Phenoxymethyl, 4-Hydroxyphenoxymethyl, α-Aminophenoxymethyl, α-Amino-4-hydroxy-phenoxymethyl, Methoxymethyl, tert.-Butoxymethyl, Thienyloxymethyl, α-Aminothienyloxymethyl, Furyloxymethyl und α-Aminofuryloxymethyl.

Für gegebenenfalls substituiertes Arylthioalkyl, Heteroarylthioalkyl und Alkylthioalkyl stehen die obengenannten Bedeutungen, die im Alkylteil oder zwischen Alkyl- und Aryl- bzw. Heteroarylteil eine Schwefelbrücke in Form einer Thioetherfunktion tragen. Besonders erwähnt seien: Phenylthiomethyl, 4-Hydroxyphenylthiomethyl, α-Aminophenylthiomethyl, 2-Methyl-1-thia-3,4-diazol-5-yl-thiomethyl, Methylthiomethyl und tert.-Butylthiomethyl.

Gegebenenfalls substituiertes Alkoxy bzw. Aryloxy steht für oben definierte Alkyl- bzw. Arylreste, welche über eine Sauerstoffbrücke direkt gebunden sind. Besonders erwähnt seien: Methoxy, Ethoxy, tert.-Butoxy, Phenoxy, Benzyloxy, Diphenylmethyloxy, 4-Nitrobenzyloxy und 4-Methoxybenzyloxy.

Gegebenenfalls substituiertes Alkylthio bzw. Arylthio steht für oben definierte Alkyl- bzw. Arylreste welche über eine Schwefelbrücke direkt gebunden sind. Besonders erwähnt seien: Methylthio, Ethylthio, tert.-Butylthio, Phenylthio, Benzylthio, Diphenylmethylthio und 4-Nitrobenzylthio.

Als erfindungsgemässe neue Oxazolinoazetidinone der Formel (1) seien beispielhaft die nachfolgenden Verbindungen genannt

| Verbindung (1) | Rest R |
|---|---|
| I | H |
| II | $CH_3$ |
| III | $C(CH_3)_3$ |
| IV | 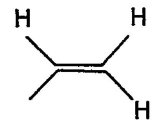 |
| V | Ph |
| VI | 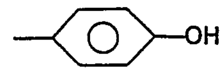 |
| VII | 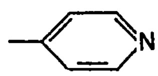 |
| VIII | $-OC(CH_3)_3$ |
| IX | 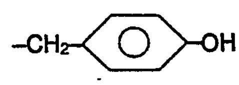 |
| X | 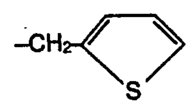 |
| XI | 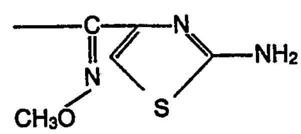 |
| XII | $-CH_2-O-Ph$ |
| XIII | 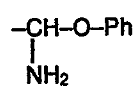 |
| XIV | 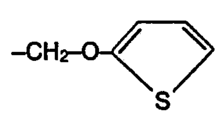 |
| XV | $-CH_2-O-CH_3$ |

| Verbindung | Rest R |
|---|---|
| XVI | 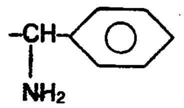 |
| XVII | 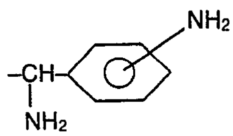 |
| XVIII | 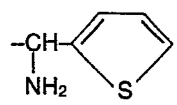 |
| XIX | 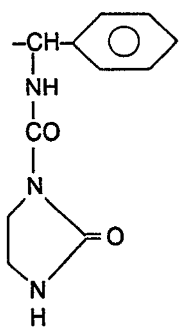 |
| XX | 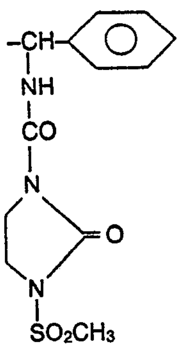 |
| XXI | $-CH_2-S-Ph$ |
| XXII | 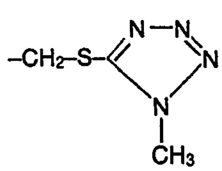 |
| XXIII | 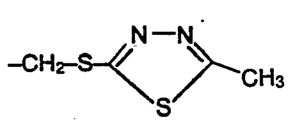 |
| XXIV | $-CH_2-S-CH_3$ |
| XXV | $-SC(CH_3)_3$ |

Bei der Umsetzung von Verbindungen der Formel (4) zu Verbindungen der Formel (2) kommen als Reagentien alle üblichen organischen und anorganischen Basen in Betracht. Hierzu gehören vorzugsweise die Alkalihydroxide, Alkalicarbonate, Alkaliamide und organische Amine. Besonders geeignet sind Kaliumcarbonat, Triethylamin, Diisopropylethylamin, Pyridin, Dimethylanilin, Diethylamin, 1,5-Diazabicyclo(5,4,0)undec-5-en (DBU), bzw. DBN und Ammoniak.

Als Verdünnungsmittel kommen alle inerten organischen Lösungsmittel sowie organische Basen und Wasser in Betracht. Hierzu gehören vorzugsweise Ethylacetat, Tetrahydrofuran, Dichlormethan, Dichlorethan, Dichlorbenzol, Toluol, Ethylamin und Dimethylamin. Die Isomerisierung erfolgt im allgemeinen bei Temperaturen zwischen $-50$ und $+50\,°C$, vorzugsweise jedoch zwischen $0\,°C$ und Raumtemperatur.

Bei der Umsetzung von Verbindungen der Formel (2) zu Verbindungen der Formel (3) kommen als Reagentien alle üblichen Oxidationsmittel in Betracht, die eine olefinische Doppelbindung in der angegebenen Weise zu spalten vermögen.

Vorzugsweise seien Natriumperiodat, Osmiumtetroxid, Sauerstoff-Ozon sowie deren Gemische genannt. Als Verdünnungsmittel kommen alle inertem organischen Lösungsmittel in Frage. Hierzu gehören vorzugsweise Ethylacetat, Ethanol, Methanol, Tetrahydrofuran, Dichlormethan, Dichlorethan, Toluol und Dioxan bzw. Gemische derselben.

Gegebenenfalls ist bei diesem Reaktionsschritt vor der Aufarbeitung ein Reduktionsmittel zuzusetzen. Vorzugsweise können dafür anorganische bzw. organische Schwefelverbindungen eingesetzt werden. Besonders geeignet sind Diorganylsulfide wie z.B. Dimethylsulfid.

Die Umsetzung erfolgt im allgemeinen zwischen $-80\,°C$ und $+50\,°C$ bevorzugt jedoch zwischen $-80\,°C$ und $0\,°C$.

Bei der Umsetzung von Verbindungen der Formel (2) zu den erfindungsgemässen Verbindungen der Formel (1) kommen als Reagentien alle üblichen Oxidationsmittel in Frage. Bevorzugt finden solche Verwendung, die zunächst zu einer Dihydroxylierung der konjugierten Doppelbindung führen. Die erwähnte Umsetzung kann gegebenenfalls in Analogie zu bekannten Verfahren durchgeführt werden (E.G. Brain et al., J.C.S. Chem. Comm. (1972) 229, Deutsche Offenlegungsschrift 2 156 352, A.K. Bose et al., Tetrahedron 37, 2321 (1981), J.S. Wells et al., J. Antibiot. 35 189 (1982)).

Bevorzugte Oxidationsmittel sind Kaliumpermanganat, Natriumperiodat, Osmiumtetroxid und deren Gemische.

Ebenso können Oxidationsmittel wie Bleitetraacetat, Kupfer(II)acetat oder N-Halogensuccinimide und -phthalimide verwendet werden. Als Lösungsmittel kommen sämtliche Lösungsmittel in Frage, welche solvolytisch wirksam sind und die derartige Ablösung des oxidierten Butenoat-Restes von β-Lactam bewirken. Im besonderen seien genannt: Wasser, Methanol, Ethanol, Aceton, Pyridin, Triethylamin, DMF oder Gemische derselben. Gegebenenfalls können basische oder saure Hilfsmittel verwendet werden. Hierzu gehören vorzugsweise Kaliumcarbonat, Puffer-Lösungen, organische Amine wie Triethylamin oder Pyridin, Schwefelsäure, Kieselsäure bzw. Kieselgel, oder organische Sulfonsäuren. Die Umsetzung erfolgt bevorzugt zwischen −40 °C und +80 °C.

Bei der Umsetzung von Verbindungen der Formel (3) zu den erfindungsgemässen Verbindungen der Formel (1) kommen als Verdünnungsmittel sämtliche solvolytisch wirksamen Lösungsmittel in Frage, die geeignet sind die Solvolyse der Oxamidstruktur zu bewirken, wie sie analog beispielsweise in der Europäischen Patentschrift 21 676, der Deutschen Offenlegungsschrift 2 839 646 und bei R.D.G. Cooper et al., J. Am. Chem. Soc. 94, 1021 (1972) beschrieben ist.

Bevorzugt gehören hierzu organische Alkohole, primäre Amine und Wasser bzw. deren Mischungen mit inerten Lösungsmitteln. Besonders erwähnt seien Methanol, und weitere Alkohole mit 1–5 C-Atomen. Gegebenenfalls können zur Unterstützung der Umsetzung basische oder saure Hilfsmittel zugesetzt werden. Hierzu gehören vorzugsweise Alkalialkoholate, Alkalicarbonate, Pufferlösungen, organische Amine, Carbonsäuren, Sulfonsäuren und anorganische Protonensäuren. Besonders seien erwähnt: Natriummethanolat, Kaliumcarbonat, schwach basische bzw. schwach saure Pufferlösungen, Schwefelsäure, Perchlorsäure, Phosphorsäure, Kieselsäure und Kieselgel. Die Umsetzung erfolgt bevorzugt zwischen −30 °C und +70 °C besonders jedoch zwischen 0 °C und Raumtemperatur.

Beispiele

Beispiel 1

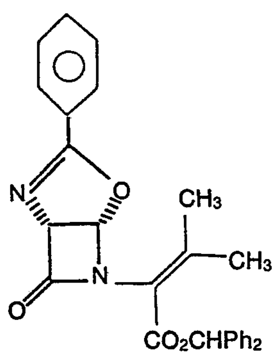

Eine Lösung von 4,53 g (10,0 mmol) Diphenylmethyl-2-[(1R,5S)-3-phenyl-7-oxo-4-oxa-2,6-diazabicyclo[3.2.0]hept-2-en-6-yl]-3-methylbut-3-enoat in 250 ml wasserfreiem Dichlormethan wurde mit 558 µl (4,0 mmol) Triethylamin versetzt und 6 h bei Raumtemperatur gerührt. Anschliessend wurde in 300 ml eiskalte 1 N HCl gegossen, mit 2 × 50 ml Dichlormethan extrahiert, mit 200 ml gesättigter $NaHCO_3$-Lösung und Wasser gewaschen und über $MgSO_4$ getrocknet. Nach Abdampfen des Lösungsmittels im Vakuum und Chromatographie des Rückstandes an 300 g Kieselgel (Toluol:Ethylacetat 85:15) erhielt man 3,81 g (84%) Diphenylmethyl-2-[(1R,5S)-3-phenyl-7-oxo-4-oxa-2,6-diazabicyclo[3.2.0]hept-2-en-6-yl]-3-methylbut-2-enoat als farblosen Hartschaum, Rf 0,54 (Ether).

IR (KBr): 1783 (C=O, β-Lactam), 1722 (C=O, Ester), 1632 $cm^{-1}$ (C=N).

$^1H$-NMR (200 MHz, $CDCl_3$): δ = 1,83 (s, 3H, $CH_3$), 2,28 (s, 3H, $CH_3$), 5,40 (s, J=4 Hz; 1H, H-5), 6,08 (d, J=4 Hz; 1H, H-1), 6,95 (s, 1H, $COOCHPh_2$), 7,3-7,6 (m, 13H, $C_6H_5$), 7,95 (m, 2H, o-Phenyl-H).

$C_{28}H_{24}N_2O_4$ (452,5)

| | | | |
|---|---|---|---|
| Berechnet | C 74,32 | H 5,32 | N 6,19 |
| Gefunden | C 74,0 | H 5,3 | N 6,2 |

Beispiel 2

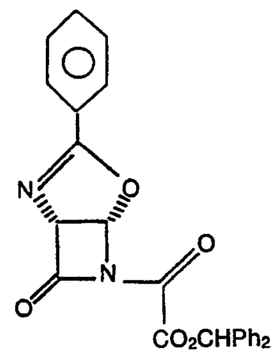

Durch eine auf −70 °C gekühlte Lösung von 1,50 g (3,32 mmol) Diphenylmethyl-2-[(1R,5S)-3-phenyl-7-oxo-4-oxa-2,6-diazabicyclo[3.2.0]hept-2-en-6-yl)]-3-methyl-2-enoat in 50 ml wasserfreiem Dichlormethan wurde bis zur Blaufärbung ein Ozon-Sauerstoffgemisch geleitet. Anschliessend wurde 10 min. mit Stickstoff gespült um überschüssiges Ozon zu entfernen, dann wurden bei −70 °C 1,95 ml (26,52 mmol) Dimethylsulfid zugegeben. Es wurde 30 min. bei −10 °C und 1 h bei Raumtemperatur gerührt und dann das Lösungsmittel im Vakuum abgedampft. Der Rückstand wurde in 100 ml Dichlormethan aufgenommen, mit gesättigter $NaHCO_3$-Lösung und Wasser gewaschen und über $MgSO_4$ getrocknet. Die organische Phase wurde im Vakuum eingeengt, mit etwas Chloroform aufgenommen mit Ether versetzt und stehen gelassen. Man erhielt 989 mg (70%) Diphenylmethyl-2-[(R1,5S)-3-phenyl-7-oxo-4-oxa-2,6-diazabicyclo[3.2.0]hept-2-en-6-yl]-2-oxo-acetat als farblose Kristalle, Schmp. 182 °C, Rf 0,37 (Ethylacetat:Hexan 1:1) – [Zersetzt sich bei Kontakt zu Kieselgel] – $[\alpha]_D^{20}$: 35,5° (1,006% in Chloroform).

IR (KBr): 1817 (C=O, β-Lactam), 1754 (C=O, Ester), 1712 (C=O, Amid), 1640 $cm^{-1}$ (C=N).

$^1H$-NMR (200 MHz, $CDCl_3$): δ = 5,55 (d, J=4,5 Hz, 1H, H-5), 6,54 (d, J=4,5 Hz, 1H, H-1), 7,07 (s, 1H, $COOCHPh_2$), 7,2-7,6 (m, 13H, $C_6H_5$), 7,9 (m, 2H, o-Phenyl-H).

MS (70 eV): m/e = 426 ($M^+$); ber 426,4.

$C_{25}H_{18}N_2O_5$ (426,4)

| | | | |
|---|---|---|---|
| Berechnet | C 70,42 | H 4,25 | N 6,57 |
| Gefunden | C 70,4 | H 4,3 | N 6,7 |

Beispiel 3

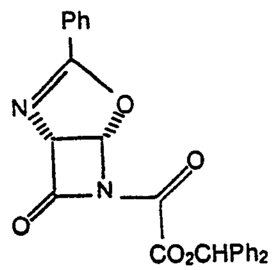

Eine Lösung von 31,68 g (70 mmol) Diphenylmethyl-2-[(1R,5S)]-3-phenyl-7-oxo-4-oxa-2,6-diazabicyclo[3.2.0]hept-2-en-6-yl]-3-methyl-3-enoat in 1,5 l wasserfreiem Dichlormethan wurde mit 3,35 ml (24 mmol) Triethylamin versetzt und 6 h bei Raumtemperatur gerührt. Danach wurde in kalte 1N HCl gegossen, mit Dichlormethan extrahiert, mit $NaHCO_3$-Lösung gewaschen und über $MgSO_4$ getrocknet. Das Trockenmittel wurde abfiltriert und die Lösung im Vakuum auf ca. 1 l eingeengt.

Man kühlte unter Stickstoffatmosphäre auf −70 °C und leitete bis zur Blaufärbung ein Ozon-Sauerstoffgemisch ein. Anschliessend wurde 10 min. mit Stickstoff gespült um überschüssiges Ozon zu entfernen, dann wurden bei −70 °C 41 ml (559 mmol) Dimethylsulfid zugegeben. Es wurde 30 min. bei −10 °C und 1 h bei Raumtemperatur gerührt und dann das Lösungsmittel im Vakuum abgedampft.

Der Rückstand wurde in 600 ml Dichlormethan gelöst, mit gesättigter $NaHCO_3$-Lösung gewaschen und über $MgSO_4$ getrocknet. Die organische Phase wurde im Vakuum eingeengt, in etwas Chloroform gelöst, mit Ether versetzt und zur Kristallisation stehen gelassen.

Man erhielt 22,3 g (70,4%) Diphenylmethyl-2-[(1R,5S)-3-phenyl-7-oxo-4-oxa-2,6-diazabicyclo-[3.2.0]hept-2-en-6-yl]-2-oxo-acetat als farblose Kristalle vom Schmp. 182 °C.

Beispiel 4

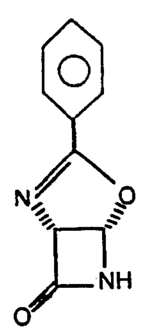

Eine Suspension von 552 mg (1,29 mmol) Diphenylmethyl-2-[(1R,5S)-3-phenyl-7-oxo-4-oxa-2,6-diazabicyclo[3.2.0]hept-2-en-6-yl]-2-oxo-acetat in 108 ml wasserfreiem Methanol wurde mit 36 ml einer 0,002%igen Lösung von Natriummethanolat in Methanol versetzt und 15 min. bei Raumtemperatur gerührt. Anschliessend gab man 21 µl Eisessig und dampfte das Methanol im Vakuum ab. Der Rückstand wurde in 100 ml Dichlormethan gelöst, mit gesättigter $NaHCO_3$-Lösung und Wasser gewaschen und über $MgSO_4$ getrocknet. Nach Abdampfen des Lösungsmittels im Vakuum und Chromatographie des Rückstands an 20 g Kieselgel (Ethylacetat:Hexan 6:4) erhielt man 112 mg (1R,5S)-3-Phenyl-7-oxo-4-oxa-2,6-diazabicyclo]3.2.0]hept-2-en als farblose Kristalle, Schmp. 159 °C, Rf 0,23 (Ether), $[\alpha]_D^{20} = -114{,}8°$ (0,836% in Aceton).

IR (KBr): 1772 (C=O, β-Lactam), 1616 $cm^{-1}$ (C=O, C=N).

$^1$H-NMR (200 MHz, DMSO): δ = 5,35 (dd, J=4Hz, J=4HZ; 1H, H-5), 6,11 (d, J=4Hz; 1H, H-1), 7,5-7,7 (m, 3H, $C_6H_5$), 7,93 (m, 2H, o-$C_6H_5$), 9,36 (d, J=4Hz; 1H, NH).

$C_{10}H_8N_2O_2$

Berechnet 188.0586

Gefunden 188.0576 (Massenspektrometr.)

Beispiel 5

Wie in Beispiel 4 beschrieben wurden aus 6,72 g (15,7 mmol) Diphenylmethyl-2-[(1R,5S)-3-phenyl-7-oxo-4-oxa-2,6-diazabicyclo[3.2.0]hept-2-en-6-yl]-2-oxo-acetat, gelöst in 750 ml wasserfreiem Methanol nach Zugabe von 250 ml 0,002% Natriummethanolat in Methanol und 18 min. bei Raumtemperatur 1,58 g (1R,5S)-3-Phenyl-7-oxo-4-oxa-2,6-diazabicyclo[3.2.0]hept-2-en erhalten. Schmp. 159–160 °C.

Beispiel 6

Eine Lösung von 426 mg (1 mmol) Diphenylmethyl-2-[(1R,5S)-3-phenyl-7-oxo-4-oxa-2,6-diazabicyclo[3.2.0]hept-2-en-6-yl]-2-oxo-acetat in 60 ml Methanol und 40 ml Dichlormethan wurde zu einer Suspension von 1 g Kieselgel in 50 ml Methanol getropft und bei Raumtemperatur gerührt. Nach Beendigung der Reaktion (DC-Kontrolle) wurde über eine Fritte mit Kieselgur abgesaugt und wie in Beispiel 4 beschrieben weiter aufgearbeitet. Man erhielt 71 mg (1R,5S)-3-Phenyl-7-oxo-4-oxa-2,6-diazabicyclo[3.2.0]hept-2-en als farblose Kristalle. Schmp. 157 °C.

Beispiel 7

Eine Lösung von 452 mg (1 mmol) Diphenylmethyl-2-[(1R,5S)-3-phenyl-7-oxo-4-oxa-2,6-diazabicyclo[3.2.0]hept-2-en-6-yl]-3-methylbut-2-enoat in 25 ml Aceton wurde innerhalb von 30 min. bei 0 °C mit einer Lösung von 297 mg (1,9 mmol) Kaliumpermanganat in 7 ml Wasser und 4,5 ml Phosphatpuffer (pH 7) versetzt.

Es wurde weitere 30 min. bei 0 °C gerührt, dann gab man 100 ml Ethylacetat zu, dampfte das Aceton im Vakuum ab, gab 200 ml gesättigte NaCl-Lösung zu und saugte über Kieselgur ab. Die organische Phase wurde mit gesättigter $NaHCO_3$-Lösung und Wasser gewaschen und über $MgSO_4$ getrocknet. Die Reinigung erfolgt wie in Beispiel 4 beschrieben. Man erhielt 36 mg (19%) (1R,5S)-3-Phenyl-7-oxo-4-oxa-2,6-diazabicyclo[3.2.0]hept-2-en als farblose Kristalle, Schmp. 158–159 °C.

Beispiel 8

Eine Lösung von 4,26 g (10 mmol) Diphenylmethyl-2-[(1R,5S)-3-phenyl-7-oxo-4-oxa-2,6-diazabicyclo[3.2.0]hept-2-en-6-yl]-2-oxo-acetat in 70 ml Dichlormethan wurde zu einer Suspension von 5 g Kieselgel in 200 ml Methanol gegeben und 8 min. bei Raumtemperatur gerührt. Danach wurde abgesaugt, das Methanol i.Vak. entfernt und der Rückstand in Dichlormethan gelöst. Es wurde mit gesättigter $NaHCO_3$-Lösung und Wasser gewaschen und über $MgSO_4$ getrocknet. Nach Abdampfen des Lösungsmittels und Chromatographie des Rückstands an 250 g Kieselgel (Hexan:Ethylacetat 2:3) erhielt man 1,17 g (62%) (1R,5S)-3-Phenyl-7-oxo-4-oxa-2,6-diazabicyclo[3.2.0]hept-2-en als farblose Kristalle.

Beispiel 9

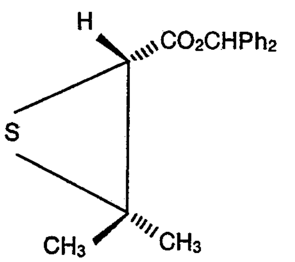

Wie bei R.J. Stoodley et al. J.C.S. Perkin I (1979), 1852 beschrieben, wurden 3,73 g (25 mmol) L (+)-penicillamin (Aldrich) mit 125 ml 0,2n HCl bei 0 °C behandelt. Dabei wurden 1,26 g (38%) (R)-3,3-dimethylthiiran-2-carbonsäure als Sirup erhalten. Dieses Material wurde in 100 ml trockenem THF gelöst und portionsweise mit frisch bereitetem Diphenyldiazomethan behandelt bis eine purpurne Farbe blieb. Nachdem das Lösungsmittel abgedampft war, wurde der Rückstand durch Blitzchromatographie über 275 g Silicagel (Toluol/Ethylacetat) gereinigt. So wurden 2,59 g (86%) Diphenylmethyl-(2R)-3,3-dimethylthiiran-2-carboxylate als Öl erhalten.

IR (unverdünnt) 1730–1740 $cm^{-1}$ (C=O Ester)

$^1$H-NMR (200 MHz, $CDCl_3$) δ: 1.68 (s, 3H, $CH_3$), 1.71 (s, 3H, $CH_3$), 3.35 (s, 1H, H-2), 6.93 (s, 1H, $COOCHPh_2$), 7.3-7.6 (m, 1OH, Ph).

MS (70 eV): m/e 315 ($M^+$); berechnet 315.4.

Beispiel 10

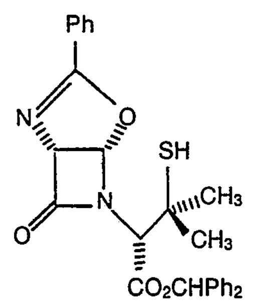

Zu einer Lösung von 546 mg (2,9 mmol) (1R,5S)-3-phenyl-7-oxo-4-oxa-2,6-diazabicyclo[3.2.0]hept-2-en und 1,01 g (3,2 mmol) Diphenylmethyl-(2R)-3,3-dimethyl-thiiran-2-carboxylat in 5,5 ml trockenem DMF wurden bei 0 °C unter Rühren 1,09 g (3,3 mmol) trockenes Caesiumcarbonat (Aldrich) gegeben. Dann wurde das Eisbad entfernt und die Lösung für 11 Stunden bei Raumtemperatur gerührt. Danach wurde sie in eine Mischung von wässriger $NaHCO_3$-Lösung und $CH_2Cl_2$ gegossen. Die organische Schicht wurde abgetrennt, mehrere Male mit Wasser gewaschen und über $MgSO_4$ getrocknet. Nach Abdampfen des Lösungsmittels im Vakuum und Chromatographie des Rückstandes über 67 g Silicagel (Toluol/Ethylacetat 3:7), wurden 470 mg (33%) Diphenylmethyl-2S-[(1R,5S)-3-phenyl-7-oxo-4-oxa-2,6-diaza-bicyclo-[3.2.0]hept-2-en-6-yl]-3-mercapto-3-methyl-butanoate als ein steifer Schaum erhalten.

IR (KBr) 1776 (C=O, β-Lactam), 1727 (C=O, Ester), 1629 $cm^{-1}$ (C=N).

$^1$H-NMR (200 MHz) δ: 1.5, 1.7 (s, je 3H, $CH_3$), 4.7 (s, 1H, CHCOOR), 5.47 (d, J=4 Hz, 1H, H-5), 6.19 (d, J=4 Hz, 1H, H-1), 6.93 (s, 1H, $CHPh_2$), 7.3-7.6 (m, 13H, Ph), 7.9 (m, 2H, o-Phenyl-H).

Beispiel 11

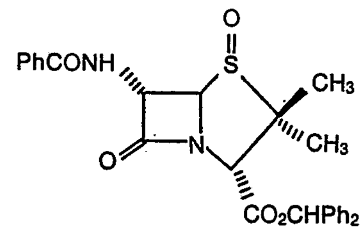

Zu 4.87 g (10 mmol) Diphenylmethyl-2S-[(1R,5S)-3-phenyl-7-oxo-4-oxa-2,6-diazabicyclo-[3.2.0]hept-2-en-6-yl]-3-mercapto-3-methyl-butanoate in 50 ml trockenem Dichlormethan wurden bei Raumtemperatur unter Rühren 86 µl (0,7 mmol) Bortrifluorid-Etherat gegeben. Die klare Lösung wurde für 4,5 Stunden bei Raumtemperatur gerührt (mit TLC-Überwachung). Dann wurden 98 µl (0,7 mmol) Triethylamin zur Neutralisierung des Lewis-Säure Katalysators zugegeben. Die Lösung wurde in einem Eisbad auf etwa +5 °C gekühlt und dann wurden 4,1 g (20 mmol) 82%ige m-Chlorperbenzoesäure auf einmal zugegeben. Die Mischung wurde für 2,5 Stunden bei +5 °C bis +10 °C gerührt und dann in eine Mischung aus 300 ml gesättigter $NaHCO_3$-Lösung und 30 ml 20%iger $Na_2SO_3$-Lösung gegossen. Die organische Phase wurde abgetrennt, mit Wasser gewaschen und über $MgSO_4$ getrocknet.

Nach Abdampfen des Lösungsmittels im Vakuum und Chromatographie des Rückstandes über 400 g Silicagel (Toluol/Ethylacetat 1:1) wurden 4.12 g (82%) Diphenylmethyl-(5R,6S)-6-benzoylamino-1-oxo-penicillan-3-carboxylat als farblose Kristalle vom Schmelzpunkt 157 bis 158 °C erhalten.

IR ($CHCl_3$) 1795 (C=O, β-Lactam), 1748 (C=O, Ester), 1672 $cm^{-1}$ (C=O, Amid).

$^1$H-NMR (200 MHz, $CDCl_3$) δ: 0,92 (s, 3H, $CH_3$), 1.64 (s, 3H, $CH_3$), 4.61 (s, 1H, CH–$CO_2$R), 5.14 (d, J=2 Hz, 1H, 5-H), 5.40 (dd, J=2 Hz, J=7 Hz, 1H,

6-H), 6.91 (s, 1H, $CHPh_2$), 7.3-7.5 (m, 13H, H arom.), 7.64 (d, J = 7 Hz, NH), 7.8 (m, 2H, orthobenzoyl-H).

$C_{28}H_{26}N_2O_5S$ (502.6)

| | | | | |
|---|---|---|---|---|
| Berechnet | C 66.9 | H 5.2 | N 5.6 | S 6.4 |
| Gefunden | C 66.6 | H 5.1 | N 5.6 | S 6.4 |

Die obengenannte Verbindung wird in bekannter Weise zu (6R,7R)-7-[2-Carboxy-2-(4-Hydroxyphenyl)acetamido]-7-methoxy-3-(1-methyl-5-tetrazolylthiomethyl)-8-oxo-5-oxa-1-azabicyclo[4.2.0]-oct-2-en-2-carbonsäure umgesetzt (M. Narisada et al. J. Antibiot. 35 463 (1982) und dort zitierte Literatur).

**Patentansprüche**

1. Verbindungen der allgemeinen Formel (1)

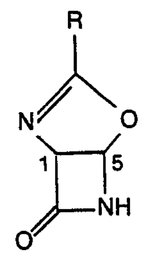

(1)

in der

R für einen in β-Lactamantibiotika üblichen Rest aus der Gruppe bestehend aus Wasserstoff, gegebenenfalls substituiertes Alkyl, Alkenyl, Alkinyl, Aralkyl mit der Ausnahme unsubstituiertes Benzyl, Aryl, Heteroaryl, Heteroaralkyl, Aryloxyalkyl, Heteroaryloxyalkyl, Akoxyalkyl, Arylthioalkyl, Heteroarylthioalkyl, Alkylthioalkyl, Alkoxy, Aryloxy, Alkylthio und Arylthio steht.

2. Verbindungen nach Anspruch 1, dadurch gekennzeichnet, dass R einen gegebenenfalls substituierten $C_1$–$C_7$-Alkylrest, einen gegebenenfalls substituierten Phenylrest, einen substituierten Benzylrest, einen gegebenenfalls substituierten Phenoxymethylrest, einen gegebenenfalls substituierten Furyl-, Thienyl-, Thiazolyl-, Isoxazolyl-, Pyridyl-, Pyrimidyl-, Pyrazolyl-, Uracyl-, Thiadiazolyl-, Tetrazolyl- oder Pyranylrest darstellt.

3. Verfahren zur Herstellung von Verbindungen der allgemeinen Formel (1)

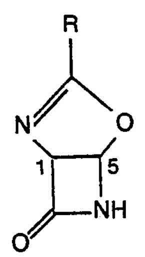

(1)

in der

R für einen in β-Lactamantibiotika üblichen Rest aus der Gruppe bestehend aus Wasserstoff, gegebenenfalls substituiertes Alkyl, Alkenyl, Alkinyl, Aralkyl mit der Ausnahme unsubstituiertes Benzyl, Aryl, Heteroaryl, Heteroaralkyl, Aryloxyalkyl, Heteroaryloxyalkyl, Alkoxyalkyl, Arylthioalkyl, Heteroarylthioalkyl, Alkylthioalkyl, Alkoxy, Aryloxy, Alkylthio und Arylthio steht, dadurch gekennzeichnet, dass man

(a) N-substituierte Oxazolinoazetidinone der allgemeinen Formel (2)

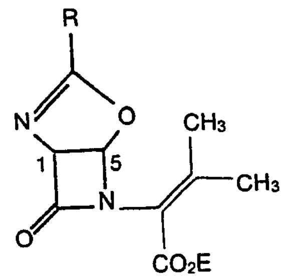

(2)

in der

R die oben angegebene Bedeutung hat und $CO_2E$ für eine Säurefunktion $CO_2H$ oder eine beliebige Esterfunktion steht, mit einem Oxidationsmittel unter solvolytischen Bedingungen in einem geeigneten Lösungsmittel gegebenenfalls in Gegenwart von säurebindenden Mitteln oder anschliessendem Einsatz von Reduktionsmitteln umsetzt, oder dass man

(b) Oxamide der allgemeinen Formel (3)

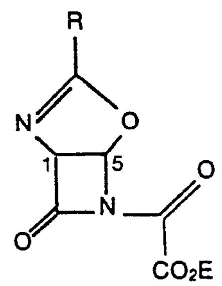

(3)

in der

R und $CO_2E$ die oben angegebene Bedeutung haben, unter solvolytischen Bedingungen in einem geeigneten Lösungsmittel gegebenenfalls in Gegenwart von Säuren und Basen umsetzt.

4. Verfahren nach Anspruch 3, dadurch gekennzeichnet, dass das Oxidationsmittel im Verfahren (a) Kaliumpermanganat, Natriumperjodat oder Osmiumtetroxid ist.

5. Verfahren nach Anspruch 3, dadurch gekennzeichnet, dass als Lösungsmittel im Verfahren (b) Wasser, primäre Amine oder Alkohole verwendet werden.

6. Verfahren nach den Ansprüchen 3 und 4, dadurch gekennzeichnet, dass die Umsetzung (a) bei −40 °C bis +80 °C durchgeführt wird.

7. Verfahren nach den Ansprüchen 3 und 5, dadurch gekennzeichnet, dass die Umsetzung (b) bei 0 °C bis Raumtemperatur durchgeführt wird.

8. Verwendung der Verbindungen gemäss Anspruch 1 zur Herstellung von β-Lactamantibiotika.

**Claims**

1. Compounds of the general formula (1)

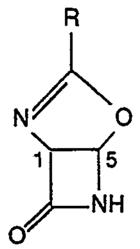

(1)

in which
R represents a radical which is customary in β-lactam antibiotics and which is from the group consisting of hydrogen or optionally substituted alkyl, alkenyl, alkynyl or aralkyl with the exception of unsubstituted benzyl, aryl, heteroaryl, heteroaralkyl, aryloxyalkyl, heteroaryloxyalkyl, alkoxyalkyl, arylthioalkyl, heteroarylthioalkyl, alkylthioalkyl, alkoxy, aryloxy, alkylthio or arylthio.

2. Compounds according to Claim 1, characterized in that R represents an optionally substituted $C_1$–$C_7$-alkyl radical, an optionally substituted phenyl radical, a substituted benzyl radical, an optionally substituted phenoxymethyl radical, or an optionally substituted furyl, thienyl, thiazolyl, isoxazolyl, pyridyl, pyrimidyl, pyrazolyl, uracyl, thiadiazolyl, tetrazolyl or pyranyl radical.

3. Process for the preparation of compounds of the general formula (1)

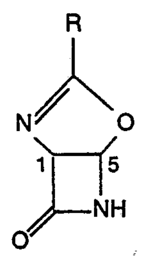

(1)

in which
R represents a radical which is customary in β-lactam antibiotics and which is from the group consisting of hydrogen or optionally substituted alkyl, alkenyl, alkynyl or aralkyl with the exception of unsubstituted benzyl, aryl, heteroaryl, heteroaralkyl, aryloxyalkyl, heteroaryloxyalkyl, alkoxyalkyl, arylthioalkyl, heteroarylthioalkyl, alkylthioalkyl, alkoxy, aryloxy, alkylthio or arylthio, characterized in that
(a) N-substituted oxazolinoazetidinones of the general formula (2)

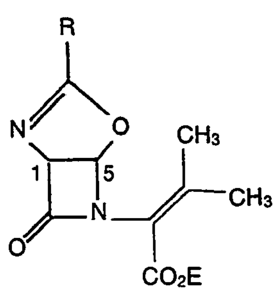

(2)

in which
R has the meaning given above and
$CO_2E$ represents an acid function $CO_2H$, or any desired ester function, are reacted with an oxidizing agent under solvolytic conditions, in a suitable solvent, if appropriate in the presence of acid-binding agents, or with subsequent use of reducing agents, or in that
(b) oxamides of the general formula (3)

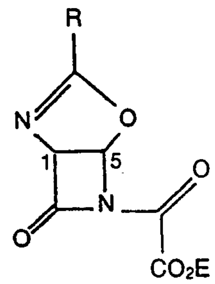

(3)

in which
R and $CO_2E$ have the meaning given above, are converted under solvolytic conditions, in a suitable solvent, if appropriate in the presence of acids or bases.

4. Process according to Claim 3, characterized in that the oxidizing agent in process (a) is potassium permanganate, sodium periodate or osmium tetroxide.

5. Process according to Claim 3, characterized in that water, primary amines or alcohols are used as solvents in process (b).

6. Process according to Claims 3 and 4, characterized in that the reaction (a) is carried out at −40 °C to +80 °C.

7. Process according to Claims 3 and 5, characterized in that the reaction (b) is carried out at 0 °C to room temperature.

8. Use of the compounds according to Claim 1 for the preparation of β-lactam antibiotics.

**Revendications**

1. Composés de formule générale (1)

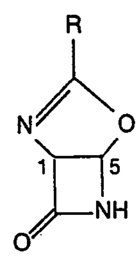

(1)

dans laquelle
R représente un reste classique dans les antibiotiques du type β-lactame, choisi dans le groupe comprenant l'hydrogène, un reste, éventuellement substitué, alkyle, alcényle, alcynyle, aralkyle, à l'exception de benzyle non substitué, aryle, hétéroaryle, hétéro-aralkyle, aryloxyalkyle, hétéro-aryloxyalkyle, alkoxyalkyle, arylthioalkyle,

hétéro-arylthioalkyle, alkylthioalkyle, alkoxy, aryloxy, alkylthio ou arylthio.

2. Composés suivant la revendication 1, caractérisés en ce que R est un reste alkyle en $C_1$ à $C_7$ éventuellement substitué, un reste phényle éventuellement substitué, un reste benzyle substitué, un reste phénoxyméthyle éventuellement substitué, un reste furyle, thiényle, thiazolyle, isoxazolyle, pyridyle, pyrimidyle, pyrazolyle, uracyle, thiadiazolyle, tétrazolyle ou pyrannyle éventuellement substitué.

3. Procédé de production de composés de formule générale (1)

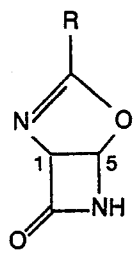

(1)

dans laquelle

R représente un reste classique dans les antibiotiques du type β-lactame, choisi dans le groupe comprenant l'hydrogène, un reste, éventuellement substitué, alkyle, alcényle, alcynyle, aralkyle, à l'exception de benzyle non substitué, aryle, hétéro-aryle, hétéro-aralkyle, aryloxyalkyle, hétéro-aryloxyalkyle, alkoxyalkyle, arylthioalkyle, hétéro-arylthioalkyle, alkylthioalkyle, alkoxy, aryloxy, alkylthio et arylthio, caractérisé en ce qu'on fait réagir

(a) des oxazolino-azétidinones substituées sur l'atome d'azote, de formule générale (2)

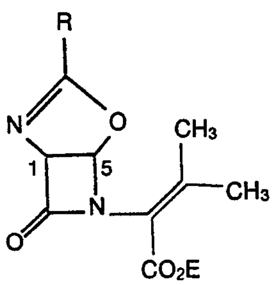

(2)

dans laquelle

R a la définition indiquée ci-dessus et

$CO_2E$ représente une fonction acide $CO_2H$ ou une fonction ester quelconque, avec un agent oxydant dans des conditions solvolytiques dans un solvant approprié, éventuellement en présence d'accepteurs d'acides ou avec l'utilisation subséquente d'agents réducteurs, ou bien

(b) des oxamides de formule générale (3)

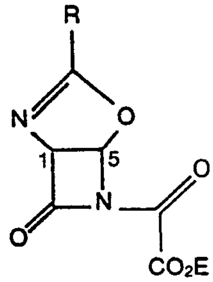

(3)

dans laquelle

R et $CO_2E$ ont la définition indiquée ci-dessus, dans des conditions solvolytiques dans un solvant approprié, le cas échéant en présence d'acides ou de bases.

4. Procédé suivant la revendication 3, caractérisé en ce que l'agent oxydant dans le procédé (a) est le permanganate de potassium, le periodate de sodium ou le tétroxyde d'osmium.

5. Procédé suivant la revendication 3, caractérisé en ce qu'on utilise comme suivant l'eau, des amines primaires ou des alcools dans le procédé (b).

6. Procédé suivant les revendications 3 et 4, caractérisé en ce qu'on conduit la réaction (a) entre −40 °C et +80 °C.

7. Procédé suivant les revendications 3 et 5, caractérisé en ce qu'on conduit la réaction (b) à une température allant de 0 °C à la température ambiante.

8. Utilisation des composés suivant la revendication 1 pour la préparation d'antibiotiques du type β-lactame.